# EUROPÄISCHE PATENTSCHRIFT

(11) **EP 1 333 756 B1**
(45) Veröffentlichungstag und Bekanntmachung des Hinweises auf die Patenterteilung: **14.12.2005**
(21) Anmeldenummer: 01982447.3
(22) Anmeldetag: 30.10.2001
(51) Int. Cl.: A61B 5/15

(54) **SYSTEM ZUR BLUTENTNAHME**
SYSTEM FOR WITHDRAWING BLOOD
SYSTEME DE PRELEVEMENT DE SANG

(30) Priorität: 31.10.2000 DE 10053974
(43) Veröffentlichungstag der Anmeldung: 13.08.2003
(60) Teilanmeldung: 05018332.6
(73) Patentinhaber: Roche Diagnostics GmbH, 68305 Mannheim (DE); F. HOFFMANN-LA ROCHE AG, 4070 Basel (CH)
(72) Erfinder: FRITZ, Michael, 68647 Biblis (DE); SACHERER, Klaus-Dieter, 67281 Kirchheim (DE); LIST, Hans, 64754 Hesseneck-Kailbach (DE); WEISS, Thomas, 68307 Mannheim (DE); DECK, Frank, 67150 Niederkirchen (DE); IMMEKUS, Claudio, 79312 Emmendingen (DE)
(86) Internationale Anmeldenummer: PCT/EP2001/012527
(87) Internationale Veröffentlichungsnummer: WO 2002/036010

(56) Entgegenhaltungen:
- EP-A- 0 565 970
- DE-A- 19 830 604
- DE-B- 2 803 345

## Beschreibung

Die vorliegende Erfindung betrifft ein System zur Entnahme von Körperflüssigkeit aus einem Körperteil, insbesondere der Fingerbeere, durch Erzeugung einer kleinen Stichwunde.

Im Bereich der klinischen Diagnostik ist es notwendig, Proben von Körperflüssigkeit, insbesondere Blutproben zu gewinnen, um darin Inhaltsstoffe nachweisen zu können. Wird eine größere Blutmenge benötigt, so wird diese im Regelfall mit einer Spritze oder dergleichen entnommen, wozu ein Blutgefäß gezielt angestochen wird. Die vorliegende Erfindung fällt jedoch in ein Gebiet, in dem lediglich Probenmengen im Bereich weniger µl oder darunter notwendig sind, um analytische Parameter zu bestimmen. Eine solche Vorgehensweise ist insbesondere zur Messung des Blutzuckerspiegels weit verbreitet, sie findet aber auch beispielsweise Anwendung um Gerinnungsparameter, Triglyzeride, HBA 1c oder Lactat zu bestimmen. Im Bereich der Diabeteserkrankung hat es sich durchgesetzt, daß Diabetiker selbst eine Überwachung des Blutzuckerspiegels vornehmen (sog. Home-Monitoring). Dies ist erforderlich, um durch gezielte Insulingaben einen Blutzuckerspiegel einzustellen, der sich im Normbereich befindet. Gerät ein Diabetiker hingegen in eine Unterzuckerung (Hypoglykämie) so kann dies eine Bewußtlosigkeit und sogar den Tod eines Patienten nach sich ziehen. Hat der Patient hingegen einen zu hohen Blutzuckerspiegel, so zieht dies gravierende Spätfolgen, wie Erblindungen und Gangräne nach sich. Für eine zur Messung des Blutzuckerspiegels erforderliche Blutentnahme haben sich kleine, handliche Blutentnahmegeräte, sog. Stechhilfen, eingebürgert, die vom Benutzer, Krankenhaus- und Pflegepersonal einfach und zuverlässig bedient werden können. Seit jüngerer Zeit sind auch Systeme zur Entnahme von interstitieller Flüssigkeit bekannt, mit der im Prinzip entsprechende Analysen durchgeführt werden können.

Ein sich in diesem Gebiet verstärkt abzeichnendes Problem besteht in der Kontamination und Verletzung durch benutzte Lanzetten. Bei vielen der im Handel befindlichen Geräten wird die Lanzette nach dem Stichvorgang entnommen oder ausgeworfen. Die in einem solchen Fall freiliegende Nadel der Lanzette kann zu Verletzungen führen, die unter Umständen Infektionen nach sich ziehen. In einigen Ländern gibt es daher bereits Bestrebungen, Blutentnahmesysteme, bei denen die Nadelspitze nach Benutzung frei zugänglich ist, zu verbieten.

In Dokumenten des Standes der Technik sind verschiedene Varianten von Blutentnahmesystemen beschrieben worden, bei denen die Nadel nach dem Stichvorgang geschützt ist. In dem Dokument US 5,314,442 wird eine Kappe beschrieben, in der eine Lanzette angeordnet ist. Zur Durchführung eines Stichvorganges wird die Lanzette durch einen Stößel oder dgl. so innerhalb der Kappe verschoben, daß die Nadel über eine Öffnung nach außen tritt. Nach dem Stich wird die Lanzette wieder ins Innere der Kappe zurückgezogen und flexible Elemente an der Lanzette sorgen dafür, daß die Lanzettennadel ohne Einwirkung des Stößels nicht mehr nach außen dringen kann. Vom Prinzip her ähnliche Systeme sind in den US Patenten 4,990,154 und 5,074,872, sowie der internationalen Anmeldung WO 00/02482 beschrieben. Ein weiteres System, bei dem das Zurückziehen einer Lanzette in eine Kappe durch eine eingebaute Feder erfolgt, ist in dem Dokument DE 198 55 465 beschrieben. Während die genannten Dokumente bereits das Problem einer Kontamination oder Verletzung des Benutzers lösen, so wird eine Ankopplung des Antriebsmechanismus an die Lanzette lediglich durch einen Preßsitz erzielt. Hierbei wird die Einstechtiefe der Nadel über einen Anschlag begrenzt. Es hat sich jedoch herausgestellt, daß das Auftreffen der Lanzette auf den Anschlag zu Vibrationen der Nadel führt, die den Schmerz beim Einstich erhöhen. Näheres zu dieser Problematik ist in EP 0 565 970 beschrieben.

Die Aufgabe der vorliegenden Erfindung bestand darin, ein System zur Entnahme von Körperflüssigkeit vorzuschlagen, das einerseits eine Kontamination oder Infektion durch benutzte Lanzetten vermeidet und andererseits dem Benutzer ein sehr schmerzarmes Einstechen ermöglicht. Eine weitere Aufgabe bestand darin, die Systeme des Standes der Technik zu vereinfachen, kostengünstiger zu machen und insbesondere ein Konzept vorzuschlagen, das verkleinert werden kann. Letzterer Punkt ist insbesondere wichtig, um ein System zur Verfügung zu stellen, das mit magazinierten Lanzetten arbeitet und dem Benutzer einen Wechsel auf eine noch unbenutzte Lanzette ermöglicht, ohne daß er hier zu komplizierte Handhabungsschritte vornehmen muß.

Die genannten Aufgaben werden durch Ausführungsformen für Systeme zur Entnahme von Körperflüssigkeit gelöst, die eine Antriebseinheit mit einem Stößel besitzen, der zur Ausführung eines Stechvorganges aus einer Ruheposition in eine Stechposition bewegt wird. Weiterhin beinhalten die Systeme eine Stecheinheit, in der sich eine Lanzette mit einer Nadel befindet, die in der Ruheposition des Stößels innerhalb der Stecheinheit angeordnet ist und die durch den Stößel bei Bewegung in die Stechposition so verschoben wird, daß die Nadel zumindest teilweise durch eine Austrittsöffnung aus der Stecheinheit austritt. Ein wesentliches Merkmal des Systems besteht darin, daß Stößel und Lanzette zur Durchführung des Stechvorganges durch Formschluß miteinander gekoppelt sind.

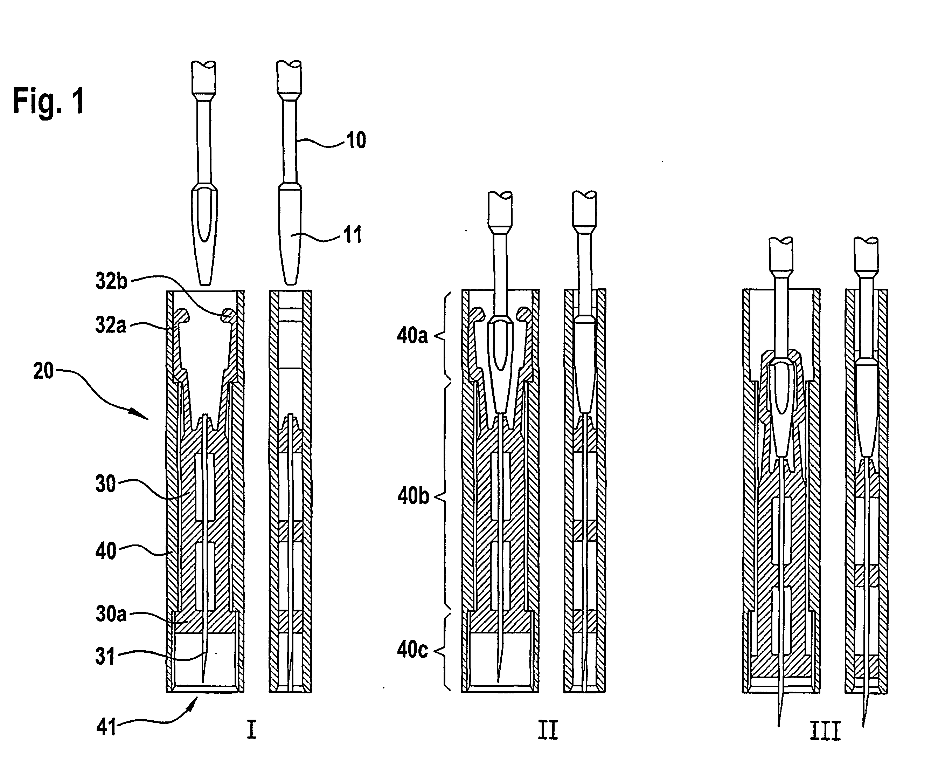

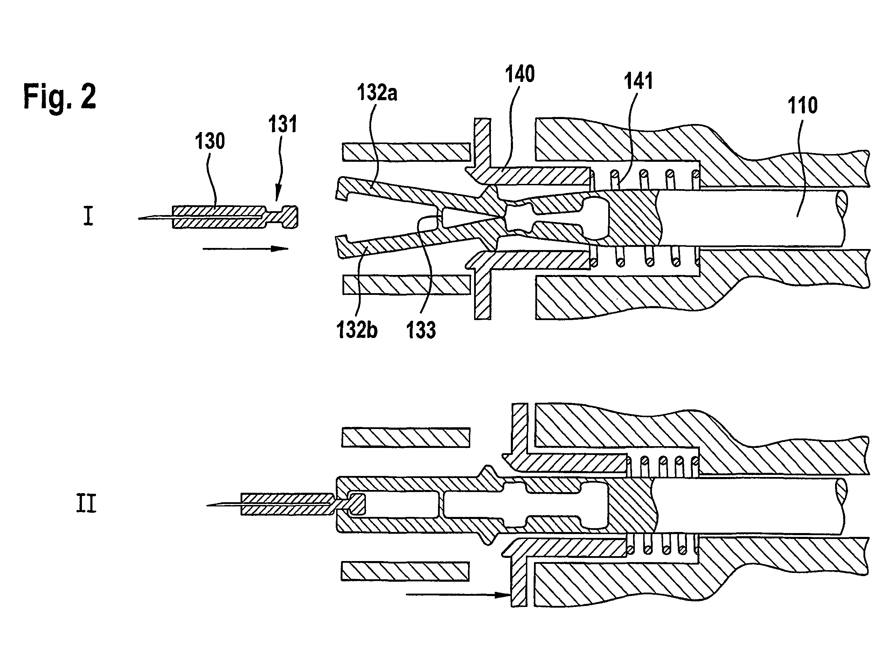

Für einen Formschluss im Sinne dieser Erfindung ist charakteristisch, dass durch ihn eine Kopplung von Lanzette und Antriebsstößel mittels eines geringen Kraftaufwandes möglich ist. Es gibt zwei prinzipielle Varianten einen Formschluss zu erzielen. Bei der ersten Variante schließt sich zur Kopplung von Lanzette und Antriebsstößel eine Form in der Weise, daß ein Halteelement umschlossen wird. Die sich schließende Form wird im Rahmen der Erfindung als Haltevorrichtung bezeichnet. Befindet sich die Haltevorrichtung an der Lanzette, so weist der Antriebsstößel ein Halteelement auf, befindet sich hingegen die Haltevorrichtung am Antriebsstößel, so ist das Halteelement an der Lanzette angeordnet. Die Figuren 1 und 2 zeigen praktische Ausgestaltungen. Vorzugsweise wird diese Variante des Formschlusses dadurch erzielt, dass durch eine Längsbewegung in Stichrichtung eine Querbewegung von Halteelementen einer (zunächst geöffneten) Haltevorrichtung erzielt wird, die sich hierbei um einen Haltebereich schließen. Vorzugsweise wid beim Schließen der Haltevorrichtung das Halteelement (zumindest teilweise) hintergriffen, so daß die Lanzette beim Zurückziehen des Antriebsstößels - zumindest bis zum etwaigen Öffnen der Haltevorrichtung - mitgenommen wird. Weiterhin ist es bevorzugt wenn Haltevorrichtung und Haltebereich geometrisch so aufeinander abgestimmt sind, daß nach erfolgtem Formschluß in Richtung der Stichbewegung keine oder nur eine geringe Toleranz besteht und die Bewegung des Antriebsstößels sowohl in Stichrichtung als auch in entgegengesetzter Richtung spielfrei in eine Bewegung der Lanzette umgesetzt wird. Dies kann erreicht werden, indem die Längsausdehnung der Kammer in der geschlossenen Haltevrrichtung mit der Längsausdehnung des Haltebereiches übereinstimmt oder nur unwesentlich größer ist (siehe Figur 1). Bei einer anderen Ausführungsform dieser Variante sind eine Ausnehmung im Haltebereich und Halteelemente an der Haltevorrichtung in ihrer Längsausdehnung so aufeinander abgestimmt, daß ein Transport in Stichrichtung im wesentlichen spielfrei möglich ist (siehe Figur 2).

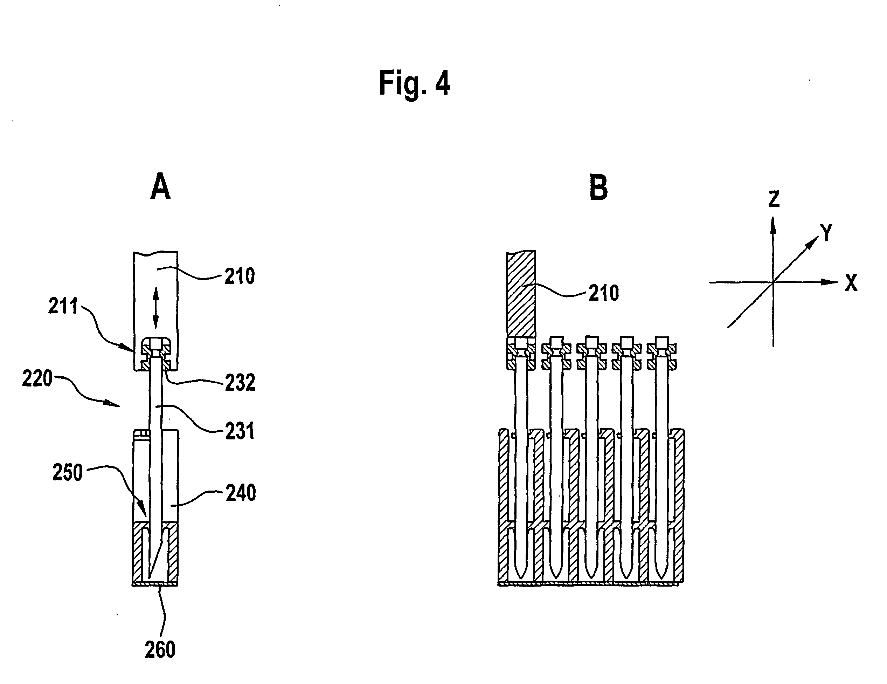

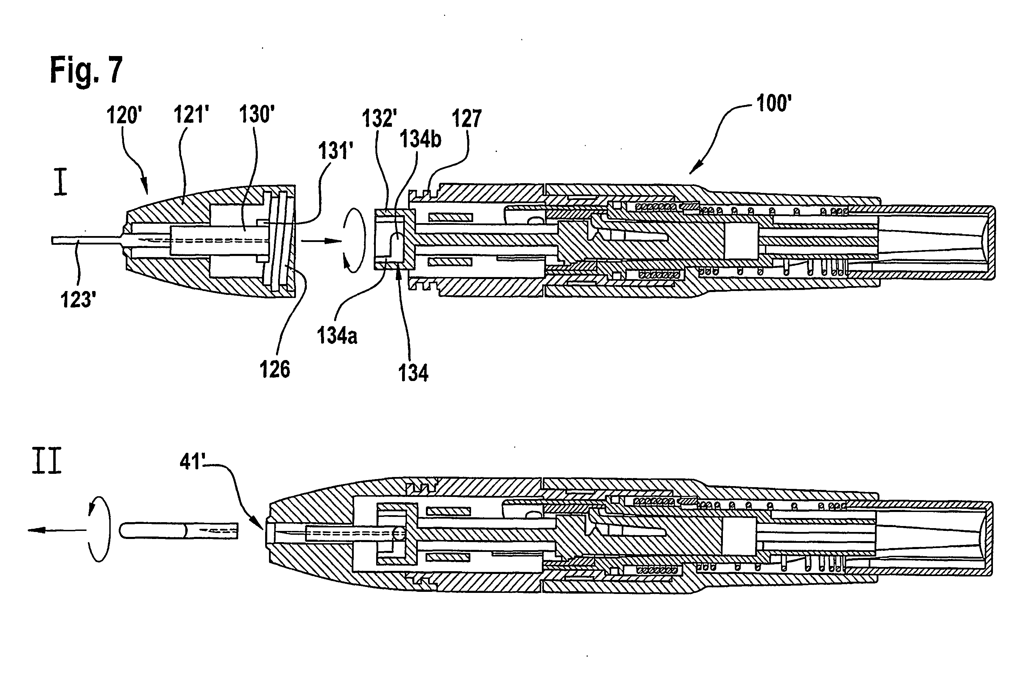

Bei einer zweiten Variante des Formschlusses sind sowohl Haltevorrichtung als auch Haltebereich im wesentlichen formstabil und ein Umschließen des Haltebereiches durch die Haltevorrichtung erfolgt durch Ineinanderbewegen der durch diese beiden Einheiten gegebenen Formen. Da die Einheiten formstabil sind, ist kein vollständiges Umschließen möglich, sondern die Formen müssen in soweit offen sein, daß sie ineinanderbewegt werden können. Das Ineinanderbewegen erfolgt dabei (zumindest mit einer Wegkomponente) quer zur Stichrichtung und es entsteht eine Kopplung, die ebenfalls im wesentlichen spielfrei in Stichrichtung ist (siehe Figur 4). Es ist schließlich auch eine Ankopplung durch eine Bewegung möglich, die sowohl Komponenten quer als auch parallel zur Stichrichtung aufweist (siehe Figur 7).

Das erfindungsgemäße System zur Blutentnahme besitzt eine Antriebseinheit mit einem Stößel, durch den eine Lanzette aus einer Ruheposition in eine Stechposition bewegt wird. Im Stand der Technik sind eine Reihe von Antriebsmechaniken bekannt, die im Gebiet der Blutentnahmegeräte eingesetzt werden können. Insbesondere werden in großem Umfang Antriebsmechaniken eingesetzt, die ihre Energie aus einer zuvor gespannten Feder beziehen. Im Rahmen der vorliegenden Erfindung werden vorzugsweise Antriebseinheiten eingesetzt, die eine geführte Bewegung des Stößels und der Lanzette, bedingt durch die formschlüssige Kopplung, ermöglichen. Mit einer geführten Bewegung ist gemeint, daß die Lanzette sowohl über einen vorbestimmten Weg in den Körper gestochen als auch über einen vorbestimmten Weg-Zeitverlauf aus dem Körper herausgezogen wird. Bei den herkömmlichen, auf einer Kombination von einer Feder und einem Anschlag basierenden System des Standes der Technik wird der Weg-Zeitverlauf durch eine Vielzahl von Parametern, wie Herstellungstoleranzen (Reibungsverhältnisse in dem System, Stärke der Feder) als auch der Hautoberfläche beeinflußt. Es hat sich gezeigt, daß eine geführte Bewegung der Lanzette, wie sie beispielsweise über eine Führungskulisse wie in der EP 565 970 beschrieben, vorteilhaft bezüglich des Einstichschmerzes ist. Bezüglich der Antriebseinheit wird hiermit auf die bevorzugten Antriebmechaniken der EP 565970 und der US 4,924,879 Bezug genommen.

Einen wichtigen Aspekt der Erfindung stellt eine von der Antriebseinheit abnehmbare Stecheinheit dar, in der sich mindestens eine Lanzette befindet. Die Stecheinheit umfaßt ein Gehäuse, in dem die Lanzette in der Ruheposition angeordnet ist. Hierdurch kann vermieden werden, daß durch die Lanzette vor oder nach Benutzung Verletzungen hervorgerufen werden bzw. daß Kontaminationen erfolgen. Das Gehäuse kann so ausgestaltet sein, daß darin eine einzelne Lanzette angeordnet ist oder das Gehäuse kann die Form eines Magazines mit mehreren Lanzetten besitzen. Im Regelfall werden sich die Lanzetten innerhalb eines Magazines in voneinander separierten Kammern befinden, um eine Kontamination ungebrauchter Lanzetten durch bereits verwendete zu verhindern. Das Gehäuse der Stecheinheit ist vorteilhaft so ausgestaltet, daß es an der Antriebseinheit angebracht werden kann. Hierzu kann die Stecheinheit beispielsweise die Form einer Kappe aufweisen, die auf die Antriebseinheit aufgesteckt wird. Derartige Ausführungsformen sind beispielsweise in den Dokumenten US 5,314,442, US 4,990,154 und US 5,074,872 beschrieben. Es sind auch Ausführungsformen möglich, bei denen die Stecheinheit fest mit der Antriebseinheit verbunden ist, beziehungsweise einen integralen Bestandteil der Antriebseinheit bildet. Dies kann insbesondere bei Systemen mit Magazin vorteilhaft sein, so daß die gesamte Einheit nach Verbrauch des Magazins weggeworfen wird. Im Falle einer Stecheinheit in Form eines Magazines kann dieses beispielsweise nebeneinander angeordnete Kammern aufweisen, in denen sich Lanzetten befinden und die Kammern nacheinander relativ zur Antriebseinheit positioniert werden, so daß die Lanzetten an den Stößel der Antriebseinheit angekoppelt werden können. Besonders vorteilhaft ist auch ein Magazin in Form einer Trommel mit parallel zur Längsachse der Trommel angeordneten Kammern, in denen sich Lanzetten befinden. Ähnlich einer Revolvertrommel kann ein solches Magazin repetierbar an der Antriebseinheit angebracht sein.

Eine weitere Anforderung an die Stecheinheit liegt in der Sterilität der Lanzetten, die über einen langen Zeitraum gewährleistet sein muß. Eine Sterilität der Stecheinheit kann, wie im Stand der Technik üblich, durch Gammabestrahlung erzielt werden. Zum Aufrechterhalten der Sterilbedingungen kann die Stecheinheit in eine Umverpackung, beispielsweise einen Polyäthylenbeutel eingeschweißt werden. In einer anderen Ausführungsform werden die Öffnungen der Stecheinheit (zum Eintritt des Stößels und für den Austritt der Nadelspitze) durch Siegelfolien verschlossen. Dies können beispielsweise abziehbare Siegelfolien sein, die der Benutzer vor Benutzung entfernt. Vorteilhaft können jedoch auch dünne Folien verwendet werden, die bei der Benutzung von dem Stößel bzw. von der Nadelspitze durchstoßen werden, so daß dem Benutzer zusätzliche Handhabungsschritte erspart bleiben. Solche Folien können bereits integral im Herstellungsprozeß der Stecheinheit, der in der Regel ein Spritzgußprozeß ist, erzeugt werden.

Bei einer weiteren, vorteilhaften Ausführungsform ist die Nadelspitze durch ein Elastomer vor Kontamination geschützt, welches vor dem Stich abgezogen wird oder während des Stichvorganges durchstochen wird, um die Nadelspitze freizugeben. Ein derartiger Schutz der Nadel vor Kontamination ist in der Anmeldung WO 01/66010 beschrieben auf welche hiermit Bezug genommen wird.

Innerhalb der Stecheinheit befinden sich ein oder mehrere Lanzetten mit einer Nadel. Abgesehen von etwaigen Vorrichtungen an der Lanzette, die eine formschlüssige Ankopplung an einen Stößel ermöglichen, können im Rahmen dieser Erfindung Lanzetten eingesetzt werden, wie sie im Stand der Technik hinlänglich bekannt sind. Im Regelfall besitzt eine solche Lanzette einen Grundkörper aus Kunststoff, in dem eine Metallnadel angeordnet ist. Es sind jedoch auch Lanzetten ohne einen separaten Gründkörper möglich (beispielsweise Metallnadel mit einer Verdickung an hinteren Ende als Haltebereich).

Im Rahmen der Erfindung ist es von Bedeutung, daß der Stößel der Antriebseinheit und die Lanzette zur Ausführung des Stechvorganges durch Formschluß miteinander gekoppelt werden. Hierin unterscheidet sich die Erfindung wesentlich von dem Stand der Technik, wo eine mechanische Kopplung zwischen Lanzette und Antrieb über einen Preßsitz (US 5,314,442, US 4,990,154, US 5,074,872), eine Verrastung (WO 00/02482), eine Verklemmung (US 3,030,959) oder über einen einfachen Andruck (DE 198 55465) erfolgt. Ein Formschluß ist dadurch gekennzeichnet, daß eine mechanisch zuverlässige Kopplung zwischen dem Antrieb und der Lanzette erfolgt, ohne daß hierfür eine wesentliche Andruckkraft an die Lanzette in Richtung der Stechbewegung erfolgt. Bei den Vorrichtungen des Standes der Technik, die mit einem Preßsitz arbeiten, muß in der Kappe, die die Lanzette beinhaltet, ein Federelement (z. B. DE 198 55 465) oder ein Rückhalteelement (z. B. WO 00/02482) vorgesehen werden, das so ausgelegt ist, daß die Lanzette beim Ankoppeln der Stecheinheit an die Antriebseinheit nicht aus der Kappe austritt. Federelemente in der Stecheinheit führen jedoch zu erhöhten Herstellungskosten, was besonders gravierend ist, da es sich bei der Stecheinheit um Verbrauchsmaterial handelt. Zur Überwindung eines Rückhalteelementes ist hingegen eine zusätzliche Kraft seitens der Antriebseinheit notwendig und ein Überwinden führt auch zu Vibrationen der Nadel, die sich negativ auf den Stichschmerz auswirken. Im übrigen ist bei Systemen, die mit einem Preßsitz arbeiten, eine geführte Bewegung, die ein Zurückziehen der Lanzette beinhaltet problematisch, da hierdurch der Preßsitz gelöst werden kann. Die in der WO 00/02482 beschriebene Verrasterung löst dieses Problem zwar, sie ist jedoch technisch schwer zu realisieren. Insbesondere ist es schwierig, eine solche Verrasterung in einem kontinuierlichen Herstellungsprozeß zu etablieren, da schon geringfügige Schwankungen des Materials oder der Prozeßbedingungen eine Funktionsunfähigkeit der Vorrichtung nach sich ziehen. Ein weiterer Nachteil der in der WO 00/02482 beschriebenen Vorrichtung besteht darin, daß die Verrasterung in einem Wegebereich erfolgt, der zum Einstich in den Körper dient. Die bei der Verrasterung auftretenden Kräfteschwankungen und Vibrationen wirken sich nachteilig auf den Einstichschmerz aus. Nachteilig ist bei der Vorrichtung weiterhin, daß die Nadel nach dem Einstich im Körper verbleibt und nicht aktiv zurückgezogen wird. Ein Zurückziehen der Nadel erfolgt erst beim Abnehmen der Kappe von der Antriebsmechanik. Bei einem Formschluß von Antriebsstößel und Lanzette gemäß der vorliegenden Erfindung kann hingegen eine Verbindung zwischen Stößel und Lanzette erreicht werden, ohne daß hierzu eine besondere Kraft in Richtung des Stiches aufgewendet werden müßte und darüber hinaus kann die formschlüssige Verbindung vorteilhaft dazu genutzt werden, die Nadel nach dem Einstich aktiv zurückzuziehen. Durch diese Möglichkeit zur aktiven Steuerung der Weg-Zeitkurve der Nadel über die Antriebseinheit ist es möglich, den Stechvorgang sehr schmerzarm zu gestalten.

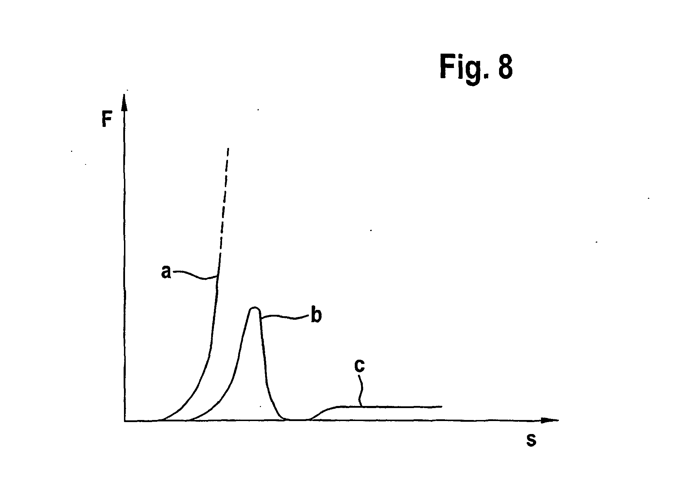

In Figur 8 sind schematische Kraft (F)-Weg (s)-Verläufe für Ankopplungsprozesse des Antriebs an die Lanzette für Preßsitz (a), Verrasterung (b) und Formschluß (c) dargestellt. Es ist zu erkennen, daß bei Verwendung des Preßsitzes die Kraft stark ansteigt, bis sich die Lanzette aus der Position, in der sie durch ein Halteelement oder eine Feder gehalten ist, löst. Bei einer Verrasterung steigt die Kraft beim Verrasten an und sinkt nach erfolgtem Schluß wieder. Bei einem Formschluß sind lediglich sehr geringe Kräfte zum Zusammenfahren der Halteelemente notwendig.

Eine weitere Eigenschaft des Formschlußes wird durch Vergleich mit dem Dokument US 3,030,959 deutlich. Bei einer Apparatur gemäß diesem US Patent werden Nadeln, die in einer Röhre angeordnet sind, nacheinander durch eine Klemmvorrichtung gehaltert, die einem Druckbleistift ähnlich ist. Neben den Kontaminationsproblemen durch gebrauchte Nadeln, die diese Apparatur ungelöst läßt, ist erkennbar, daß die Positionierung der Nadeln in axialer Richtung (d.h. in Stichrichtung) nicht definiert ist. So wie bei einem Druckbleistift die Länge der hervortretenden Bleistiftspitze vom Benutzer frei gewählt werden kann, hängt auch hier die axiale Positionierung der Nadel von der Einstellung durch den Benutzer ab. Bei einem Formschlußprinzip hingegen besitzen Lanzette und Antriebseinheit aufeinander abgestimmte Haltebereiche und Haltevorrichtungen. Durch die geometrische Ausgestaltung von Haltebereich und Haltevorrichtung kann erreicht werden, daß die axiale Positionierung der Lanzette wohl definiert ist und somit die Einstechtiefe genau kontrolliert werden kann. Durch die Verwendung eines Formschlußes kann somit sowohl eine Kraftspitze in axialer Richtung bei Kopplung von Lanzette und Antriebsstößel vermieden werden als auch eine exakte axiale Positionierung erzielt werden. Bei einem Formschluß schließt sich eine Form (Haltevorrichtung) um eine andere Form (Haltebereich). Unter einem Sich-schließen ist in diesem Sinne sowohl eine Bewegung von Vorrichtungsteilen quer zur Stichrichtung als auch alternativ ein formschlüssiges Ineinandergreifen zweier in ihrer Form unveränderter Formkörper gemeint. Bei einer bevorzugten Ausgestaltung ist die Haltevorrichtung geöffnet und schließt sich um den Haltebereich wenn dieser in die Haltevorrichtung eingeführt wird. Insbesondere kann dieses Schließen durch eine Längsbewegung erfolgen bei der die Längsbewegung in eine Bewegung von Haltelementen der Haltevorrichtung mit Komponenten quer zur Längsbewegungsrichtung umgewandelt wird. Eine praktisch vorteilhafte Möglichkeit, diese Umwandlung einer Längebewegung in eine Querbewegung zu erzielen besteht darin, die Haltevorrichtung mit der Längsbewegung in einen sich verjüngenden Kanal (z.B. in einer Hülse) einzuführen bzw. in diesem zu verschieben (siehe Figur 1). Unter sich verjüngend werden hierbei nicht nur sich kontinuierlich verjüngende Kanäle verstanden sondern allgemein Kanäle deren lichte Weite sich in der Längsrichtung verringert. Ein Kanal braucht in diesem Zusammenhang kein über eine Umfangsfläche geschlossener Körper zu sein. Wenn die Haltevorrichtung beispielsweise wie in Figur 1 zwei gegenüberliegende Haken aufweist, genügen zwei Wandungen deren innerer Abstand sich zueinander verringert.
Es besteht andererseits auch die Möglichkeit, die Haltevorrichtung durch Aufheben eines gespannten Zustandes schließen zu lassen (siehe Figur 2), was beispielsweise durch Bewegung der Haltevorrichtung in einem sich erweiternden Kanal möglich ist.

Die vorliegende Erfindung wird anhand von Figuren näher erläutert:
- Figur 1:: Querschnittsdarstellung durch eine Stecheinheit mit einer Haltevorrichtung an der Lanzette
- Figur 2:: Querschnittsdarstellung durch einen Systemausschnitt mit einer Haltevorrichtung an der Antriebseinheit
- Figur 3:: Querschnittsdarstellung durch ein Gesamtsystem und Schritte der Benutzung
- Figur 4:: Systemausschnitt mit formstabiler Haltevorrichtung an der Antriebseinheit
- Figur 5:: Magazin von Stecheinheiten
- Figur 6:: System aus Antriebseinheit und Stecheinheit
- Figur 7:: System mit einer formstabilen Haltevorrichtung
- Figur 8:: Kraft-Weg-Diagramm für verschiedene Ankopplungsarten
- Figur 9:: Stecheinheit mit Sterilschutz
- Figur 10:: Trommelförmiges Magazin

Figur 1 zeigt ein Blutentnahmesystem gemäß einer ersten Ausführungsform der Erfindung in den Betätigungsphasen I, II und III. In der Figur 1 sind lediglich Teilaspekte des Systems dargestellt. In der Darstellung fehlt die Antriebseinheit für den Stößel (10) sowie das Gehäuse der Antriebseinheit, an der die Stecheinheit (20) befestigt wird. Als Antriebseinheit für den Stößel (10) ist die in der EP 0 565 970 beschriebene Antriebsvorrichtung besonders geeignet.

Die in der Figur 1 dargestellten Phasen zeigen das Zustandekommen der formschlüssigen Kopplung zwischen dem Antriebsstößel (10) und der Lanzette (30), sowie den eigentlichen Stichvorgang. Für jede der drei Phasen sind jeweils 2 Querschnittsdarstellungen entlang der Längsachse des Systems in zueinander senkrechten Ebenen dargestellt. Aus der linken Darstellung der Phase I ist zu erkennen, daß die Lanzette (30) innerhalb einer Hülse (40) angeordnet ist. Die dargestellte Lanzette (30) besitzt einen Grundkörper, der aus Kunststoff gefertigt ist sowie eine darin eingespritzte Nadel (31) aus Stahl. An der der Nadelspitze abgewandten Seite weist die Lanzette eine Haltevorrichtung mit Halteelementen in Form zweier Haken (32a, 32b) auf. Beim Einfahren des Stößels (10) in die Lanzette gelangt ein verdickter Bereich am vorderen Ende des Stößels, der als Haltebereich (11) dient, zwischen die Haken (32a, 32b) und trifft schließlich auf das hintere Nadelende (Phase II). Es ist auch möglich, den Stößel statt auf das hintere Nadelende auf den Grundkörper der Lanzette auftreffen zu lassen. Ein direkter Kontakt mit der Nadel ist jedoch von Vorteil, da die Nadellänge produktionstechnisch sehr exakt kontrolliert werden kann und somit eine genaue Kontrolle der Einstechtiefe möglich ist. Beim weiteren Vordringen des Stößels schiebt er die Lanzette innerhalb der Hülse (40) in Richtung der Austrittsöffnung (41), so daß schließlich die Nadelspitze über die Austrittsöffnung übersteht und in ein darunter liegendes Gewebe einsticht. Aus dem Übergang von Phase II zur Phase III ist zu erkennen, daß sich die Haltevorrichtungen (32a, 32b) an der Lanzette um den Haltebereich (11) des Stößels schließen, sobald die Lanzette innerhalb der Hülse (40) verschoben wird. Die Haltevorrichtung an der Lanzette umgreift das Halteelement des Stößels so, daß eine formschlüssige Verbindung entsteht, mit der nicht nur eine Vorwärtsbewegung der Lanzette zur Ausführung eines Stiches sondern auch ein aktives, von der Antriebseinheit gesteuertes Zurückziehen der Lanzette im wesentlichen spielfrei möglich ist. Dies wird einerseits dadurch gewährleistet, daß das Ende des Haltebereiches auf dem Nadelende aufsitzt als auch durch die Haken (32a, 32b) welche das hintere Ende des Haltebereiches hintergreifen. Die Länge des Haltebereiches und die Längsausdehnung der Kammer in der geschlossenen Haltevorrichtung sind so aufeinander abgestimmt, daß ein spielfreier Antrieb der Lanzette in Stichrichtung gegeben ist. Durch die Anordnung der Lanzette bzw. der Haltevorrichtung in einer sich verjüngenden Hülse wird eine Umwandlung einer Längsbewegung der Lanzette in Stichrichtung in eine Querbewegung der Elemente der Haltevorrichtung erzielt, die einen Formschluß mit dem Haltebereich des Antriebs ermöglicht. Wie aus den Figuren zu erkennen ist, besitzt die Hülse (40) einen mittleren Bereich (40b), der gegenüber dem oberen Bereich (40a) verjüngt ist. Durch diese Verjüngung werden die Haken (32a, 32b) der Lanzette beim Verschieben der Lanzette in der Hülse in Richtung auf die Längsachse zusammengedrückt, so daß ein Umschließen des Haltebereiches (11) erfolgt. Als besonders vorteilhaft hat es sich erwiesen, die Stecheinheit (20) so auszugestalten, daß die Lanzette innerhalb der Hülse gehaltert wird, wenn keine Einwirkung des Stößels vorliegt. Hierdurch kann sichergestellt werden, daß sich die Nadel im unbetätigten Zustand innerhalb der Hülse (40) befindet und somit keine Verletzungen oder Kontamination durch eine herausragende Nadelspitze verursacht wird. Ein Hindurchrutschen der Lanzette durch die Hülse in Richtung der Austrittsöffnung (41) wird wirksam dadurch verhindert, daß die Haken (32a, 32b) einen Absatz aufweisen, der auf einer Kante des mittleren Bereiches (40b) aufliegt. Die Neigung dieser Kanten und die Flexibilität der Haken können so aneinander angepaßt werden, daß ein Einschieben in die Verjüngung einerseits mit geringer Kraft erfolgen kann und andererseits ein ungewolltes Hindurchrutschen effizient vermieden wird. Um ein Herausrutschen der Lanzette aus der Hülse, in der der Stichrichtung entgegengesetzten Richtung zu vermeiden, ist bei der dargestellten Ausführungsform ein verbreiterter Teil (40c) am unteren Ende der Hülse sowie ein damit korrespondierender verbreiterter Teil (30a) am unteren Ende des Lanzettengrundkörpers vorgesehen.

Wie aus Figur III hervorgeht, befindet sich die Nadelspitze beim Auftreffen des Haltebereiches (11) auf das Nadelende noch innerhalb der Hülse (40). Dies ist vorteilhaft, da die durch das Auftreffen erzeugte Erschütterung keinen Einfluß auf den Stichvorgang im Gewebe hat, wodurch Einstichschmerz durch eine solche Erschütterung vermieden wird.

Erfindungsgemäß ist es bevorzugt, wenn das System so ausgelegt ist, daß nach Durchlaufen der Phasen I, II und III eine Rückbewegung des Stößels (10) in umgekehrter Richtung erfolgt, so daß der Stößel von der Lanzette entkoppelt wird und sich die Lanzette wieder vollständig innerhalb der Hülse befindet. Für die Ankopplung des Stößels an die Lanzette gibt es zwei Hauptvarianten. Bei der ersten Variante sind Gehäuse, Antriebseinheit und Stecheinheit so aneinander angepaßt, daß sich der Stößel im Ausgangszustand vollständig außerhalb der Stecheinheit (20) befindet (wie für Phase I dargestellt). Ein Nachteil dieser Ausführungsform ist es, daß vom Stößel zum Durchführen eines Stiches ein verhältnismäßig langer Weg zurückgelegt werden muß. Ein Vorteil ist es jedoch, daß sich der Stößel vollständig außerhalb der Hülse befindet, so daß eine Querbewegung möglich ist. Dementsprechend kann die erste Variante vorteilhaft für Systeme mit einem Lanzettenmagazin eingesetzt werden, bei dem nacheinander verschiedene Hülsen unter den Stößel bewegt werden. Bei einer zweiten Variante wird durch Ankoppeln der Stecheinheit an die Antriebseinheit bereits eine Positionierung gemäß Phase II bzw. noch darüber hinaus in Richtung auf Phase III erreicht. Bei einer solchen Ausführungsform kann der Weg, den der Stößel vornehmen muß, sehr klein gehalten werden, was konstruktionstechnisch günstig ist.

Figur 2 zeigt eine zweite Ausführungsform der Erfindung, bei der die Lanzette (130) einen Haltebereich (131) aufweist und die Antriebseinheit eine Haltevorrichtung (132a, 132b) besitzt. In der Figur 2 ist wiederum der Bereich des Systems gezeigt, der zur Halterung der Lanzette dient, nicht jedoch die Antriebseinheit. Auch im Zusammenhang mit dieser Ausführungsform ist es vorteilhaft, eine Antriebseinheit einzusetzen, die den Stößel (110) geführt bewegt. Der Stößel trägt an seinem vorderen Ende eine Haltevorrichtung mit Halteelementen in Form zweier Haken (132a, 132b), die über eine flexible Brücke (133) (oder ein Gelenk) miteinander verbunden sind. Die Anordnung bildet ein Federelement. In der Phase I sind diese Haken gespreizt, da ihre hinteren Enden durch eine Hülse (140) zusammengehalten werden. Beim Einschieben der Lanzette (130) wird gleichzeitig die Hülse gegen eine Feder (141) verschoben, so daß die hinteren Enden der Haken freigegeben werden und sich die vorderen Enden der Haken formschlüssig um den Haltebereich (131) der Lanzette schließen. Der Haltebereich der Lanzette weist eine Ausnehmung (Vertiefungen) auf, in die die Haken der Haltevorrichtung eingreifen. Die Längsausdehnung (in Stichrichtung) der eingreifenden Enden der Haltevorrichtung und die Längsausdehnung der Ausnehmungen sind im wesentlichen gleich, so daß mit dieser Anordnung eine geführte, im wesentlichen spielfreie Stechbewegung ausgeführt werden kann.
Auch bei dieser Ausführungsform erfolgt eine Umwandlung einer Längsbewegung der Haltevorrichtung in eine Querbewegung der Halteelemente.

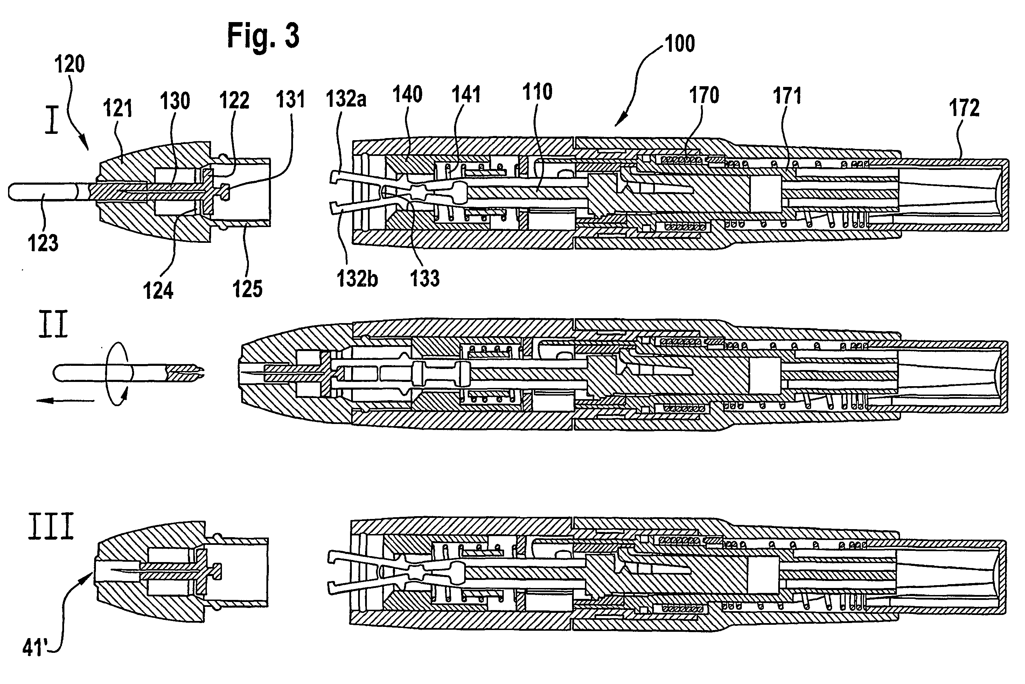

Figur 3 zeigt ein erfindungsgemäßes System, basierend auf dem formschlüssigen Ankopplungsprinzip gemäß Figur 2. Die Antriebseinheit 100 basiert auf dem Gerät Softclix®, welches in der EP B 0 565 970 beschrieben ist. Aus diesem Dokument geht insbesondere hervor, wie die durch die Antriebsfeder 170 vermittelte Drehbewegung der Hülse 171 in eine Translationsbewegung des Stößels 110 umgesetzt wird. Das Spannen der Antriebsfeder durch Niederdrücken des Druckknopfes 172 und eine hierfür geeignete mechanische Übersetzung sind in der europäischen Patentanmeldung mit dem Aktenzeichen EP 0 O10 2503.0 beschrieben. Die Antriebseinheit weist an ihrem vorderen Ende eine Haltevorrichtung mit zwei Halteelementen, im konkreten Fall Haken (132a, 132b) auf. Wie bereits zu Figur 2 ausgeführt, sind diese Haken in einem mittleren Bereich über eine flexible Brücke (133) bzw. ein Gelenk, miteinander verbunden. Auf der den Haken relativ zur Brücke (133) abgewandten Seite wird die Haltevorrichtung über eine Hülse (140) so gehaltert, daß die Haken geöffnet sind. Die Hülse (140) wird über eine in der Antriebsvorrichtung befindliche Feder (141) in Position gehalten. Aus Figur 3 I ist ferner eine Stecheinheit zu erkennen, bei der eine Lanzette (130) in einer Kappe (121) angeordnet ist. Die Lanzette besitzt an ihrem hinteren Ende einen Haltebereich (131), der von der Haltevorrichtung (132a, 132b) ergriffen wird. Der Außenkörper der Lanzette weist einen vorderen, schmalen Bereich auf sowie einen Flansch (122) zwischen diesem schmalen Bereich und dem Haltebereich (131). Die Spitze der Lanzette ist durch einen abdrehbaren Kunststoffkörper (123) vor Kontamination und mechanischen Einwirkungen geschützt. Die Kappe (121) weist in ihrem Inneren einen Durchgang für den schmaleren Bereich der Lanzette sowie einen im Querschnitt vergrößerten Bereich auf, der zur Aufnahme des Flansches (122) geeignet ist. Innerhalb dieses verbreiterten Kanales der Kappe ist ein Wulst (124) angeordnet, der ein selbständiges Hineinrutschen des Flansches in den erweiterten Kanal verhindert. Die Kappe besitzt weiterhin eine Hülse (125), welche dazu dient, die Hülse (140) der Antriebseinheit beim Aufsetzen der Stecheinheit auf die Antriebseinheit zurückzuschieben. Dieser Vorgang läßt sich durch Zusammenschau der Figuren 31 und 3II erkennen. Durch das Wegschieben der Hülse (140) mittels der Hülse (125) wird die Haltevorrichtung freigegeben, so daß sie den Haltebereich der Lanzette umfaßt, wie aus Figur 3II zu erkennen ist. Durch Drücken der Taste (172) und Abdrehen des Schutzteiles (123) von der Lanzette ist das System nunmehr für den Einsatz präpariert. Mit der in Figur 3II dargestellten Vorrichtung wird nunmehr ein Stechvorgang durchgeführt, in dem das vordere Ende der Kappe (120) auf ein Gewebeteil aufgesetzt und durch Betätigen eines Auslösemechanismus die Antriebseinheit aktiviert wird. Nach erfolgtem Stechvorgang wird die Kappe von der Antriebseinheit in Richtung der Längsachse abgezogen, wobei der Flansch (122) hinter den Wulst (124) zurückgezogen wird, so daß die kontaminierte Nadelspitze nicht mehr aus der Kappe austreten kann. Die Stecheinheit im Zustand gemäß Figur 3 III kann verworfen, oder nach Ankopplung an die Antriebseinheit für weitere Entnahmevorgänge verwendet werden.

In den nachfolgenden Figuren 4A und 4B werden weitere Beispiele einer formschlüssigen Verbindung zwischen Lanzette und Stößel gezeigt, die jedoch nicht Gegenstand der Erfindung sind.

Figur 4 zeigt eine dritte Ausführungsform der Erfindung, bei der der Formschluß zwischen Lanzette und Antrieb durch ein formschlüssiges Verbinden von geometrisch aneinander angepaßten Haltebereichen und Haltevorrichtungen erzielt wird. In der Figur 4A ist eine Stecheinheit (220) dargestellt, die eine Hülse (240) aufweist, in der sich eine Metallnadel (231) befindet. Die Hülse (240) besitzt eine dünne Querwand(250), die die Metallnadel relativ zur Hülse haltert. Diese Querwand wird vorzugsweise gleich beim Umspritzen der Nadeln mit Kunststoff geformt. Aufgrund der relativ geringen Dicke dieser Wand kann die mechanische Verbindung von Hülse und Nadel beim Stichvorgang gelöst werden, so daß die Nadel an der Wand (250) vorbeigleitet. An der Austrittsöffnung der Hülse ist diese mit einer dünnen Folie (260) verschlossen, die beim Stichvorgang durchstochen wird. An ihrem oberen Ende trägt die Nadel (231) einen angespritzten Haltebereich (232). Zur mechanischen Stabilisierung weist die Nadel eine Verjüngung auf, um die der Haltebereich (232) gespritzt wird, so daß ein axiales Hindurchrutschen vermieden wird. Eine Halterung der Nadel in der Hülse kann auch durch Aufrauhung der Nadel an ihrer Außenfläche, einer Verdickung oder Krümmung der Nadel im Bereich der Hülse erzielt werden. Der Antriebsstößel (210) dieser Ausführungsform weist an seinem unteren Ende eine Haltevorrichtung (211) auf, die den Haltebereich (232), wie dargestellt, formschlüssig umfaßt. Die Haltevorrichtung (211) ist seitlich offen, so daß der Stößel parallel versetzt zur Nadel mit der Haltevorrichtung (211) auf die Höhe des Haltebereiches (232) gefahren werden und durch Verschiebung quer zur Nadelachse bzw. zur Stichrichtung mit dem Haltebereich der Nadel in Eingriff gebracht werden kann. Nach diesem somit erzielten Formschluß kann die Nadel sowohl in Stichrichtung von dem Stößel (210) angetrieben werden als auch aktiv zurückgezogen werden. Im dargestellten Beispiel weist der Haltebereich der Lanzette eine Ausnehmung (Vertiefung) auf, in die bei Ankopplung ein Vorsprung der Haltevorrichtung eingreift, so daß die beiden Körper in Bezug auf die Stichrichtung weitestgehend spielfrei miteinander verbunden sind.

In der Figur 4B ist ein Magazin dargestellt, welches aus Stecheinheiten (220) gemäß Figur 4A aufgebaut ist. Bezogen auf das dargestellte Koordinatensystem kann der Antriebsstößel (210) durch Bewegung in Y-Richtung (senkrecht zur Zeichenebene) mit dem Haltebereich der Lanzette in Eingriff gebracht werden bzw. der Formschluß auch wieder aufgehoben werden. Bei aufgehobenem Formschluß kann der Antriebsstößel in X-Richtung (rechts / links) auf Höhe einer anderen Lanzette bewegt und durch Bewegung in Y-Richtung wiederum mit dieser in Eingriff gebracht werden, so daß die Lanzetten des Magazins sukzessive abgearbeitet werden können. Bei bestehendem Formschluß ist eine aktive Bewegung der Nadeln sowohl in positiver als auch negativer Z-Richtung (oben / unten) möglich.

Alternativ zu der Ankopplung gemäß Figur 4B, die sowohl eine Bewegung des Antriebsstößels in x als auch in γ-Richtung erfordert, kann auch eine Anlcopplungsbewegung über eine Bewegung nur in x-Richtung erfolgen. Der Antriebsstößel weist hierzu beispielsweise zwei gegenüberliegende Haken auf, zwischen denen in x-Richtung ein Durchgang für den Haltebereich der Lanzette vorgesehen ist. Durch Bewegung in x-Richtung kann der Stößel nunmehr von Lanzette zu Lanzette bewegt werden, um einen Stichvorgang in z-Richtung auszuführen. Befindet sich der Antriebsstößel in x-Richtung auf Höhe einer Lanzette, so umfaßt die Haltevorrichtung des Stößels den Haltebereich der Lanzette formschlüssig und diese kann durch den Stößel in z-Richtung geführt bewegt werden, der Stößel führt demnach eine Stichbewegung aus und zieht auch die Lanzette wieder aktiv zurück.

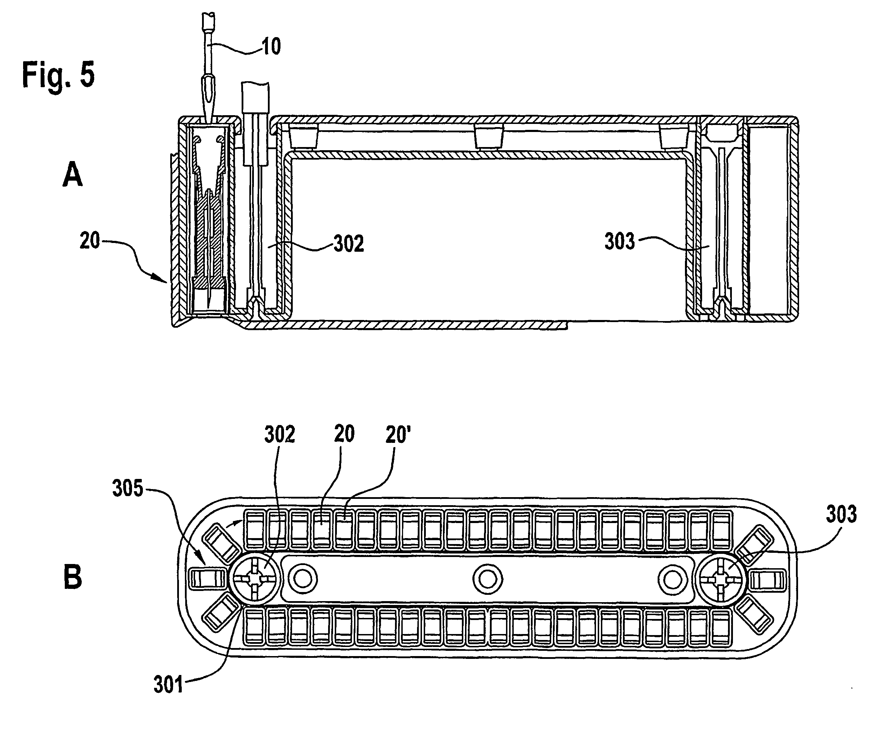

In Figur 5 ist ein automatisch arbeitendes System mit Stecheinheiten gemäß Figur 1 dargestellt. Wie aus der Aufsicht (Figur 5B) zu erkennen ist, sind die Stecheinheiten (20, 20' etc) nebeneinander an einem Transportband befestigt. Das Transportband (301) läuft um zwei voneinander beabstandete Walzen (302, 303). Eine der Walzen wird durch eine Motor angetrieben, so daß die Stecheinheiten sukzessive durch eine Ankopplungsposition (305) hindurchbewegt werden. In dieser Position ist, wie in Figur 4a dargestellt, eine formschlüssige Ankopplung eines Antriebsstößels (10) an eine in der Stechposition (305) befindliche Stecheinheit (20) möglich.

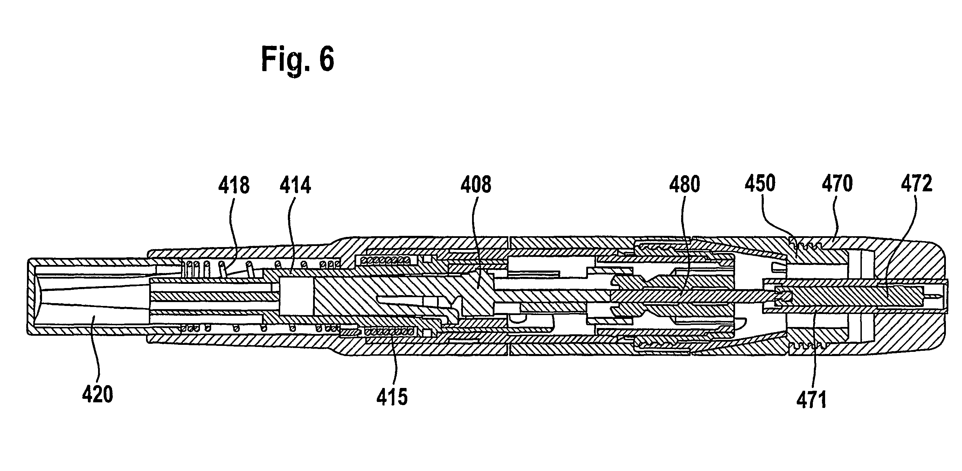

Figur 6 zeigt eine Antriebseinheit, an die eine Stecheinheit analog der Ausführungsform in Figur 1 angekoppelt ist. Das dargestellte Antriebssystem entspricht dem der europäischen Patentanmeldung Aktenzeichen 00102503.0. Bei diesem Antriebssystem wird durch das Drücken eines Druckknopfes (420) entgegen der Spannung einer Feder (418) eine Hülse (414) axial gedreht, so daß eine zweite Feder (415) gespannt wird. Die Hülse (414) wird in einer Endposition arretiert, so daß die zweite Feder (415) gespannt bleibt. Wenn der Benutzer die Arretierung aufhebt, entspannt sich die Feder (415) und die Hülse (414) wird in entgegengesetzte Richtung wie beim Spannvorgang gedreht. In der Hülse (414) befindet sich eine Nut, die als Führungskulisse für den Vortriebszylinder (408) dient, der auf seiner Außenfläche einen Zapfen oder dergleichen trägt, welcher in die Nut eingreift. Die Rotation der Hülse (414) wird so in eine Translation des Vortriebszylinders umgesetzt. Der Vortriebszylinder überträgt seinen Vortrieb auf den Antriebsstößel (480), der an seinem vorderen Ende einen Haltebereich aufweist.

Die Antriebseinheit weist weiterhin einen Haltebereich (450) auf, auf den eine Stecheinheit aufgesteckt oder aufgedreht werden kann. Die Stecheinheit beinhaltet eine Kappe (470), die eine Fläche zum Andrücken an die Hautoberfläche besitzt. In der Kappe ist eine Hülse (471) angeordnet, in welcher eine Lanzette (472) mit Haltevorrichtungen an ihrer, der Nadelspitze abgewandten Seite, aufweist. Die Haltevorrichtung der Lanzette entspricht den Haltevorrichtungen (32a, 32b) der Figur 1. Aus Figur 6 ist weiterhin zu erkennen, daß eine formschlüssige Verbindung von Lanzette und Antriebsstößel bereits durch Anbringen der Kappe (470) an der Antriebsvorrichtung erzielt wird.

Figur 7 zeigt ein System zur Entnahme von Körperflüssigkeiten, das eine Vielzahl von Ähnlichkeiten zu dem in Figur 3 dargestellten System aufweist. Insbesondere wird auf die bei Figur 3 und Figur 6 erfolgte Beschreibung des Antriebes und Spannmechanismus verwiesen. Das System gemäß Figur 7 weist eine Stecheinheit (120') mit einer Kappe (121') und einer Lanzette (130') auf. In der Kappe (121') befindet sich ein axialer Durchgang, durch den der Lanzettenkörper beim Stechvorgang hindurchtreten kann. Vorzugsweise sind sowohl Durchgang als auch Lanzettenkörper so aufeinander abgestimmt, daß eine axiale Führung der Lanzette beim Stechvorgang mit lediglich geringem Spiel in Querrichtung gegeben ist. An ihrem hinteren Ende weist die Kappe ein Gewinde (126) auf, das auf ein entsprechendes Gewinde (127) der Antriebseinheit (100') aufgeschraubt werden kann. An dem der Nadelspitze entgegengesetzten Ende besitzt die Lanzette einen oder auch mehrere (im dargestellten Fall 2) Zapfen (131'), die beim Aufsetzen bzw. Aufdrehen der Kappe auf die Antriebseinheit in Formschluß mit der Haltevorrichtung (132') gelangen. Die Haltevorrichtung weist hierzu eine Ausnehmung oder Nut auf, die einen axialen Teil (134a) sowie einen quer dazu angeordneten Teil (134b) aufweist. Beim Aufsetzen der Kappe auf die Antriebseinheit gelangen die Zapfen (131') in den axialen Teil der Nut (134) und durchfahren diesen bis auf Höhe des quer angeordneten Teiles der Nut. Durch Aufdrehen der Kappe (120') auf die Antriebseinheit (100') wird der Zapfen (131') vom Ende des axialen Teils in dem Querteil der Nut bis zum gegenüberliegenden Ende verfahren. Wie aus Figur 7II zu erkennen ist, wird die Lanzette mittels der Zapfen durch die Haltevorrichtung (132') axial gehaltert, so daß eine geführte Stechbewegung mit der Lanzette erfolgen kann. Durch die Lagerung der Zapfen im Querteil der Nuten kann mit der Lanzette sowohl eine Bewegung zum Austreten der Nadelspitze als auch ein Zurückziehen erfolgen. Wie aus Figur 7 ersichtlich ist, kann die formschlüssige Verbindung zwischen Lanzette und Haltevorrichtung erzielt werden, ohne daß ein Verklemmen oder Verrasten erfolgt. Das in Figur 7 dargestellte Kopplungsprinzip von Lanzette und Antriebsstößel ist selbstverständlich auch reziprok, d. h. mit einer entsprechenden Haltevorrichtung an der Lanzette und einem Haltebereich an dem Stößel bzw. Antrieb möglich.

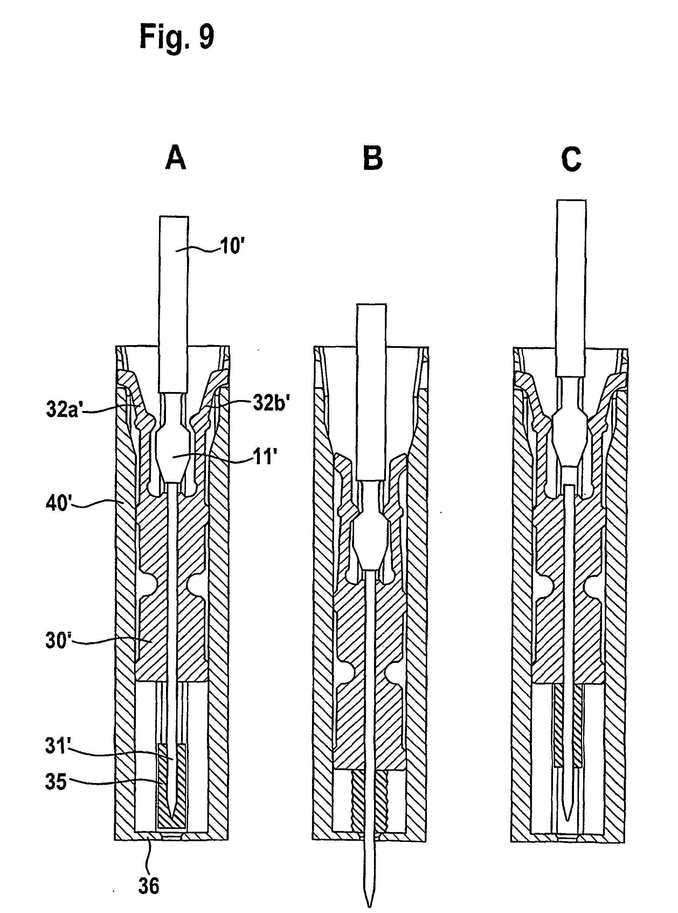

Figur 9 zeigt eine Weiterentwicklung des Systems aus Figur 1. Der Antriebsstößel (10') weist einen Haltebereich (11') auf, der an seiner Oberseite eine umlaufende, schräge Fläche besitzt, die, wie in Figur 9B zu erkennen, beim Stechvorgang in Passung mit schrägen Flächen der Haken (32a', 32b') kommt. Das Ende des Antriebsstößels sitzt auf dem Nadelende auf, während das Zusammendrücken der in sich flexiblen Haken (32a', 32b') bewirkt, daß Schrägen an der Hakeninnenseite an Schrägen an der Oberseite des Haltebereiches gedrückt werden, so daß der Haltebereich auf das Nadelende gedrückt wird und eine in Stichrichtung spielfreie Verbindung von Haltebereich und Lanzette entsteht. Durch diese Passung kann eine sehr präzise formschlüssige Halterung zwischen Lanzette und Antriebsstößel erreicht werden, die (Herstellungs-)Toleranzen ausgleicht, so daß sowohl beim Stich als auch bei der Rückziehbewegung ein Spiel vermieden wird.
Die Haltevorrichtung in Form der Haken (32a', 32b') ist ferner so gestaltet, daß die freien Enden der Haken in Ausnehmungen in der Hülse (40') eingreifen. Hierdurch wird vermieden, daß die Lanzette (30') aus der Hülse (40') ungewollt herausrutschen kann. Wie aus dem Übergang von Figur 9A zu Figur 9B zu erkennen ist, werden die freien Enden der Haken beim Beginn des Stechvorganges zunächst aus den Ausnehmungen gelöst und die Haken werden beim Einschieben in die sich verjüngende Hülse zusammengedrückt, so daß sie den Haltebereich des Antriebsstößels umschließen.

In der Figur 9 ist weiterhin zu erkennen, daß die Nadelspitze (31') in einem Material (35) angeordnet ist. Dieses Material (35) ist vorzugsweise ein Elastomer, das die Nadelspitze dicht umgibt, so daß eine Kontamination der Nadelspitze wirksam verändert wird. Als geeignete Elastomere kommen in Frage: Styrol-Oligoblock-Copolymere, thermoplastische Polyolefine, thermoplastische Polyurethane, thermoplastische Copolyester und thermoplastische Copolyamide. Bezüglich weiterer Details zu dieser Vorgehensweise, eine Kontamination der Lanzettenspitze zu vermeiden, wird hiermit auf die internationale Anmeldung WO 01/66010 Bezug genommen. In der Ausgangsposition gemäß Figur A vor dem Stich, befindet sich die Nadelspitze in dem Elastomer und wird beim Durchführen eines Stiches von der Nadel durchstochen, wie aus Figur B zu erkennen. Hierzu weist die Hülse (40') an ihrer Unterseite eine Platte (36) mit zentraler Öffnung auf. Die Platte verhindert ein Austreten des Elastomers durch die Öffnung, so daß dieses durchstochen wird, während die Nadel durch die zentrale Öffnung austritt. Beim Zurückziehen der Lanzette verbleibt das Elastomer an der Nadel, wobei jetzt die Nadelspitze frei liegt (siehe Figur C).

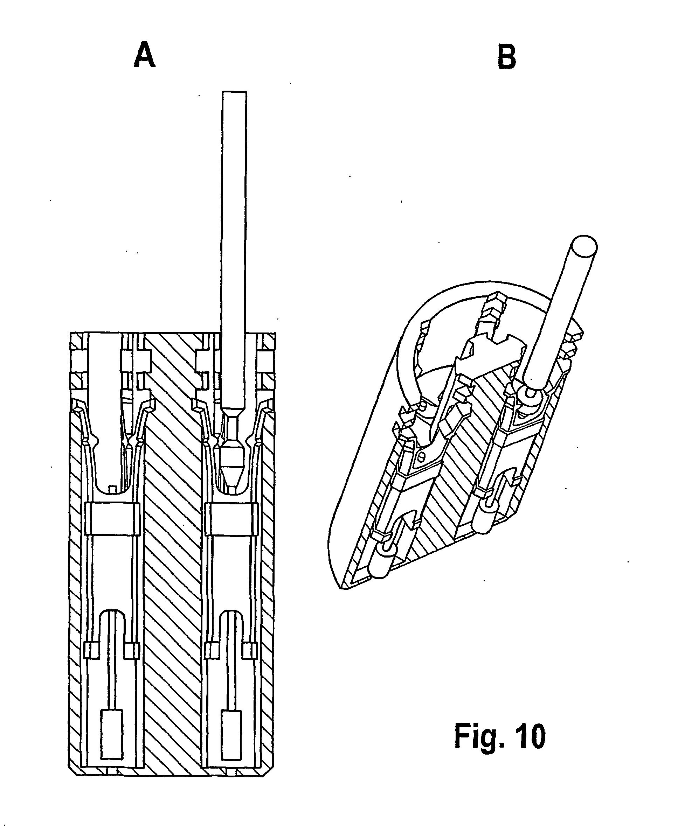

Figur 10 zeigt im Querschnitt (Figur A) und in perspektivischer Ansicht (Figur B) ein zylindrisches Magazin auf Basis von Lanzetten gemäß Figur 1 bzw. Figur 9. Mit einem solchen Magazin ist es möglich, auf einfache Weise neue Lanzetten an den Antrieb anzukoppeln. Hierzu kann beispielsweise der Antriebsstößel ortsfest bezüglich einer Stechhilfe angeordnet sein und das in Figur 10 darstellte, trommelförmige Magazin wird ähnlich einer Revolvertrommel gedreht, so daß ungebrauchte Lanzetten in die Position zur Ankopplung des Antriebsstößels gelangen.

## Patentansprüche

1. System zur Entnahme von Körperflüssigkeit, beinhaltend
eine Antriebseinheit (100, 100') mit einem Stößel (10, 110), der zur Ausführung eines Stechvorganges aus einer Ruheposition in eine Stechposition bewegt wird, sowie
eine Stecheinheit (20, 120, 120'), in der sich eine Lanzette (30, 130, 130') mit einer Nadel befindet, die in der Ruheposition des Stößels innerhalb der Stecheinheit angeordnet ist und die durch den Stößel bei Bewegung in die Stechposition so verschoben wird, dass die Nadel zumindest teilweise durch eine Austrittsöffnung (41, 41') in der Stecheinheit austritt,
wobei Stößel und Lanzette zur Ausführung des Stechvorganges durch Formschluss miteinander gekoppelt sind, und der Formschluss **dadurch** erzielt wird, dass eine Haltevorrichtung (132a, 132b/32a, 32b) des Stößels oder der Lanzette sich um einen Haltebereich (11, 131) des Stößels oder der Lanzette schließt und die Haltevorrichtung der Lanzette oder des Stößels mindestens ein bewegliches Element (32a, 32b/132a, 132b) aufweist, **dadurch gekennzeichnet, dass** sich das mindestens eine bewegliche Element in einer offenen Position befindet, wenn die Lanzette und der Stößel nicht miteinander gekoppelt sind und aufgrund einer Längsbewegung der Lanzette das mindestens eine bewegliche Element eine Bewegung quer zur Einstichrichtung ausführt, so dass das mindestens eine bewegliche Element sich um den Haltebereich schließt, wobei eine formschlüssige Kopplung von Stößel und Lanzette erfolgt.

2. System gemäß einem der Ansprüche 1, bei dem die Stecheinheit eine Hülse (40) beinhaltet, in der die Lanzette verschiebbar angeordnet ist.

3. System gemäß Anspruch 2, bei dem die Hülse einen Kanal aufweist, in dem die Lanzette bewegt wird und der Kanal eine Verjüngung aufweist, durch die bei Bewegung der Lanzette in Richtung auf die Stechposition mindestens ein bewegliches Element quer zu dieser Richtung bewegt wird.

4. System gemäß einem der vorangehenden Ansprüche, bei dem die Stecheinheit abnehmbar an der Antriebseinheit befestigt ist.

5. System gemäß Anspruch 4, bei dem eine formschlüssige Ankopplung des Stößels an die Lanzette bei Anbringen der Kappe an der Antriebseinheit erfolgt.

6. System gemäß Anspruch 4 oder 5, bei dem durch Abnehmen der Stecheinheit von der Antriebseinheit eine Entkopplung von Stößel und Lanzette erfolgt.

7. System gemäß Anspruch 1, bei dem die Haltevorrichtung mindestens zwei flexible Elemente aufweist, die sich bei Ankopplung des Stößels an der Lanzette aufeinander zubewegen.

8. System gemäß Anspruch 1, das eine Einstellvorrichtung für die Austrittsweite der Nadel aus der Kappe besitzt.

9. System gemäß Anspruch 1, das ein Magazin mit einer Mehrzahl von Lanzetten besitzt, die nacheinander an den Stößel der Antriebseinheit angekoppelt werden können.

10. System gemäß Anspruch 1, bei dem die Haltevorrichtung zwei oder mehr Halteelemente (132a, 132b) aufweist, die sich beim Aufsetzen der Stecheinheit auf die Antriebseinheit schließen, so dass die Halteelemente den Haltebereich der Lanzette haltern.

11. System gemäß Anspruch 10, bei dem die Halteelemente mit einem Federelement verbunden sind, das die Halteelemente aufeinander zubewegt.

12. System gemäß Anspruch 11, bei dem die Halteelemente durch ein Spannelement gegen die Kraft der Federelemente gespannt werden können, so dass sie geöffnet sind und ein Aufsetzen der Stecheinheit auf die Antriebseinheit das Spannelement löst, so dass sich die Halteelemente aufeinander zubewegen.

13. System gemäß Anspruch 1, bei dem sich eine Haltevorrichtung an der Lanzette oder am Antriebsstößel befindet, die mindestens eine Nut (134a) in axialer Richtung der Lanzette aufweist, welche an ihrem einen Ende offen ist, so daß ein Einführen von einem oder mehreren Zapfen an der Lanzette oder dem Antriebsstößel möglich ist und die Nut (134b) am anderen Ende in eine Nut quer zur axialen Richtung übergeht.

14. Stecheinheit (20) zum Anbringen an eine Antriebseinheit, beinhaltend eine Lanzette (30) mit einer Nadel und einer Haltevorrichtung mit Halteelementen (32a, 32b) zur Herstellung einer formschlüssigen Verbindung mit einem Stößel der Antriebseinheit, wobei die Haltevorrichtung zunächst geöffnet ist und beim Antreiben der Lanzette der Stößel in die Haltevorrichtung einkoppeln kann, wobei sich die Halteelemente aufgrund einer Längsbewegung der Lanzette quer zur Längsrichtung bewegen, so dass eine formschlüssige Kopplung zwischen der Stecheinheit und Stößel erfolgen kann.

15. Stecheinheit gemäß Anspruch 14 mit einer Hülse (40), in der die Lanzette angeordnet ist,
wobei
die Hülse eine Verjüngung aufweist, durch die die Haltevorrichtung beim Verschieben der Lanzette in der Hülse zumindest teilweise geschlossen wird.

## Claims

1. System for withdrawing body fluid comprising
a drive unit (100, 100') having a plunger (10, 110) which is moved from a resting position into a lancing position in order to carry out a lancing process and
a lancing unit (20, 120, 120') containing a lancet (30, 130, 130') with a needle which in the resting position of the plunger is arranged within the lancing unit and is displaced by the plunger when it moves into the lancing position in such a manner that the needle at least partially emerges through an exit opening (41, 41') in the lancing unit,
the plunger and lancet being coupled together by a form fit in order to carry out a lancing process, and the form fit being achieved by means of a holding device (132a, 132b/32a, 32b) of the plunger or the lancet enclosing a holding area (11, 131) of the plunger or of the lancet, and the holding device of the lancet or of the plunger having at least one movable element (32a, 32b/132a, 132b), **characterized in that** the at least one movable element is in an open position when the lancet and plunger are not coupled together and as a result of a longitudinal movement of the lancet the at least one movable element executes a movement transversely to the lancing direction in such a manner that the at least one movable element encloses the holding area to make a form-fitting coupling between the plunger and lancet.

2. System as claimed in claim 1, in which the lancing unit contains a sleeve (40) in which the lancet is movably located.

3. System as claimed in claim 2, in which the sleeve has a channel in which the lancet is moved and the channel has a taper by means of which the at least one movable element is moved at right angles to this direction when the lancet is moved towards the lancing position.

4. System as claimed in one of the previous claims, in which the lancing unit is detachably attached to the drive unit.

5. System as claimed in claim 4, in which the plunger is coupled to the lancet in a form-fitting manner when the cap is attached to the drive unit.

6. System as claimed in claim 4 or 5, in which the plunger and lancet are disconnected when the lancing unit is removed from the drive unit.

7. System as claimed in claim 1, in which the holding device has at least two flexible elements which move towards one another when the plunger is coupled to the lancet.

8. System as claimed in claim 1, which has a device for adjusting the extent to which the needle emerges from the cap.

9. System as claimed in claim 1, which has a magazine containing a plurality of lancets that can be successively coupled to the plunger of the drive unit.

10. System as claimed in claim 1, in which the holding device has two or more holding elements (132a, 132b) which close when the lancing unit is placed on the drive unit such that the holding elements hold the holding area of the lancet.

11. System as claimed in claim 10, in which the holding elements are connected by a spring element which moves the holding elements towards one another.

12. System as claimed in claim 11, in which the holding elements can be tensioned by a tensioning element against the force of the spring elements so that they are opened and placing the lancing unit on the drive unit releases the tensioning element such that the holding elements move towards one another.

13. System as claimed in claim 1, in which a holding device is located on the lancet or on the driving plunger which has at least one groove (134a) in the axial direction of the lancet which is open at one end to enable insertion of one or more pins on the lancet or on the driving plunger and the other end of the groove (134b) merges into a groove at right angles to the axial direction.

14. Lancing unit (20) for attachment to a drive unit comprising a lancet (30) having a needle and a holding device with holding elements (32a, 32b) to make a form-fitting connection with a plunger of the drive unit in which the holding device is firstly opened and the plunger can connect into the holding device when the lancet is propelled during which the holding elements are moved at right angles to the longitudinal direction due to a longitudinal movement of the lancet thus enabling the lancing unit and plunger to couple in a form-fitting manner.

15. Lancing unit as claimed in claim 14 with a sleeve (40) in which the lancet is arranged wherein
the sleeve has a taper by means of which the holding device is at least partly closed when the lancet is inserted into the sleeve.

## Revendications

1. Système pour le prélèvement d'un fluide corporel, contenant
une unité d'entraînement (100, 100') avec un poussoir (10, 110) qui est soumis à un mouvement depuis une position de repos jusque dans une position de piqûre pour la mise en oeuvre d'un processus de piqûre, et
une unité de piqûre (20, 120, 120') dans laquelle se trouve une lancette (30, 130, 130') comprenant une aiguille qui est disposée à l'intérieur de l'unité de piqûre, dans la position de repos du poussoir et qui est déplacée par le poussoir lors du mouvement dans la position de piqûre, de telle sorte que l'aiguille sort, au moins en partie à travers une ouverture de sortie (41, 41') dans l'unité de piqûre,
le poussoir et la lancette pour la mise en oeuvre du processus de piqûre étant accouplés l'un à l'autre par conjugaison de forme, la conjugaison de forme étant obtenue de telle sorte qu'un dispositif de retenue (132a, 132b/32a, 32b) du poussoir ou de la lancette se referme autour d'une zone de retenue (11, 131) du poussoir ou de la lancette et le dispositif de retenue de la lancette ou du poussoir présente au moins un élément mobile (32a, 32b/132a, 132b), **caractérisé en ce que** ledit au moins un élément mobile se trouve dans une position ouverte lorsque la lancette et le poussoir ne sont pas accouplés l'un à l'autre, et sur base d'un mouvement longitudinal de la lancette ledit au moins un élément mobile effectue un mouvement transversalement par rapport à la direction de piqûre, de telle sorte que ledit au moins un élément mobile se referme autour de la zone de retenue, si bien que l'on obtient un accouplement par conjugaison de forme entre le poussoir et la lancette.

2. Système selon la revendication 1, dans lequel l'unité de piqûre contient un manchon (40) dans lequel est disposée la lancette de manière à pouvoir s'y déplacer.

3. Système selon la revendication 2, dans lequel le manchon présente un canal dans lequel se déplace la lancette et le canal présente un rétrécissement à travers lequel, lors de la mise en mouvement de la lancette dans la direction de la position de piqûre, au moins un élément mobile est mis en mouvement en position transversale par rapport à cette direction.

4. Système selon l'une quelconque des revendications précédentes, dans lequel l'unité de piqûre est fixée de manière amovible à l'unité d'entraînement.

5. Système selon la revendication 4, dans lequel on obtient un accouplement par conjugaison de forme du poussoir à la lancette lors de l'application du capuchon sur l'unité d'entraînement.

6. Système selon la revendication 4 ou 5, dans lequel, lors du retrait de l'unité de piqûre de l'unité d'entraînement, on obtient un désaccouplement entre poussoir et lancette.

7. Système selon la revendication 1, dans lequel le dispositif de retenue présente au moins deux éléments flexibles qui se déplacent l'un vers l'autre lors de l'accouplement du poussoir à la lancette.

8. Système selon la revendication 1, qui possède un dispositif de réglage pour l'étendue de sortie de l'aiguille à l'extérieur du capuchon.

9. Système selon la revendication 1, qui possède un magasin comprenant une multitude de lancettes qui peuvent être accouplées de manière successive au poussoir de l'unité d'entraînement.

10. Système selon la revendication 1, dans lequel le dispositif de retenue présente deux ou plusieurs éléments de retenue (132a, 132b) qui, lors de l'application de l'unité de piqûre, se raccordent à l'unité d'entraînement, si bien que les éléments de retenue retiennent la zone de retenue de la lancette.

11. Système selon la revendication 10, dans lequel les éléments de retenue sont reliés à un élément de ressort qui déplace les éléments de retenue l'un vers l'autre.

12. Système selon la revendication 11, dans lequel les éléments de retenue peuvent être serrés via un élément de serrage à l'encontre de la force exercée par les éléments de ressort, si bien qu'ils sont ouverts et une application de l'unité de piqûre sur l'unité d'entraînement libère l'élément de serrage, si bien que les éléments de retenue se déplacent l'un vers l'autre.

13. Système selon la revendication 1, dans lequel un dispositif de retenue est placé sur la lancette ou sur le poussoir d'entraînement, qui présente au moins une rainure (134a) dans la direction axiale de la lancette, qui est ouverte à une de ses extrémités, de telle sorte que l'on peut introduire un ou plusieurs tourillons sur la lancette ou sur le poussoir d'entraînement et que la rainure (134b) se transforme à l'autre extrémité en une rainure transversale par rapport à la direction axiale.

14. Unité de piqûre (20) destinée à venir s'appliquer sur une unité d'entraînement, contenant une lancette (30) comprenant une aiguille et un dispositif de retenue comprenant des éléments de retenue (32a, 32b) pour l'obtention d'une liaison par conjugaison de forme avec un poussoir de l'unité d'entraînement, le dispositif de retenue étant ouvert dans un premier temps, le poussoir étant en mesure de s'accoupler dans le dispositif de retenue, lors de l'entraînement de la lancette, les éléments de retenue, en raison d'un mouvement longitudinal de la lancette, se déplaçant transversalement par rapport à la direction longitudinale, si bien que l'on peut obtenir un accouplement par conjugaison de forme entre l'unité de piqûre et le poussoir.

15. Unité de piqûre selon la revendication 14, comprenant un manchon (40) dans lequel est disposée la lancette, le manchon présentant un rétrécissement à travers lequel le dispositif de retenue, lors du déplacement de la lancette dans le manchon, se ferme au moins en partie.
